# EUROPÄISCHE PATENTSCHRIFT

(11) **EP 0 400 168 B1**
(45) Veröffentlichungstag und Bekanntmachung des Hinweises auf die Patenterteilung: **14.09.1994**
(21) Anmeldenummer: 89109729.7
(22) Anmeldetag: 30.05.1989
(51) Int. Cl.: A61B 17/22

(54) **Medizintechnische Anlage zur Positionierung eines Therapiegerätes**
Medical installation to position a therapeutical apparatus
Installation médicale pour positionner un appareil thérapeutique

(43) Veröffentlichungstag der Anmeldung: 05.12.1990
(73) Patentinhaber: SIEMENS AKTIENGESELLSCHAFT, 80333 München (DE)
(72) Erfinder: Hüttenrauch, Gerd, Dipl.-Ing. (FH), D-8525 Uttenreuth (DE); Seubert, Hans-Peter, Dipl.-Ing. (FH), D-8551 Heroldsbach (DE); Seyler, Gerhard, Dipl.-Ing. (FH), D-8521 Bubenreuth (DE)

(56) Entgegenhaltungen:
- EP-A- 0 269 801
- EP-A- 0 288 698
- EP-A- 0 390 653
- WO-A-90/05485
- DE-A- 3 736 733

## Beschreibung

Die Erfindung betrifft eine medizintechnische Anlage gemäß dem ersten Teil des Patentanspruches 1.

Eine medizintechnische Anlage dieser Art kann beispielsweise als Therapiegerät einen Stoßwellengenerator für die Zertrümmerung von Körpersteinen aufweisen. Ein Stoßwellengenerator dieser Art sendet Stoßwellen aus, die auf einen Fokusbereich fokussiert sind. Es ist dabei erforderlich, den Stoßwellengenerator für die Behandlung so zu Positionieren, daß der Fokusbereich mit dem jeweils zu zertrümmernden Körperstein zusammenfällt.

Durch die EP-A-0 269 801 ist eine Lithotripsieanlage bekannt, bei der Informationen über die räumliche Lage des Konkrementes mit Hilfe einer Röntgenanlage gewonnen werden, durch die zwei Röntgenbilder aus unterschiedlichen Richtungen erzeugt werden. Diese Informationen werden durch ein Röntgensystem mit zwei Monitoren gebildet, auf denen die Bilder des Konkrementes erscheinen, die unter verschiedenen Richtungen aufgenommen worden sind. Anhand der Monitorbilder wird über einen Lichtgriffel die Istposition des Konkrementes in einen Rechner eingegeben, der bewirkt, daß das Konkrement durch Verstellung des Patiententisches in den Fokus eines Stoßwellengenerators bewegt wird.

Die Verwendung nur eines Röntgenfilmes, auf dem die aus verschiedenen Richtungen aufgenommenen Bilder eines Konkrementes erscheinen, ist aus den unter **Artikel 54(3) EPÜ** zu berücksichtigenden Dokumenten WO-A-9005485 und EP-A-0390653 bekannt; die räumlichen Koordinaten des Konkrementes werden hier aus dem Abstand der Projektionen, dem Schwenkwinkel der Aufnahmen und weiteren geometrischen Kenngrössen der Anlage wie dem Abstand zwischen Film und Röntgenkamera bestimmt.

Der Erfindung liegt die Aufgabe zugrunde, eine medizintechnische Anlage der eingangs genannten Art gegenüber dem Stand der Technik insbesondere hinsichtlich der Ausbildung des Ortungssystems zu vereinfachen, wobei der Patient bei der Einstelung in Ruhe bleiben soll.

Diese Aufgabe ist erfindungsgemäß gelöst durch die Merkmale des zweiten Teiles des Patentanspruches 1. Bei Durchstrahlung des Objektes mit einer Röntgeneinheit unter zwei verschiedenen Richtungen lassen sich Informationen über die räumliche Lage eines Körpersteines erhalten. Diese Informationen können in den Rechner eingegeben werden, der das Therapiegerät so nachführt, daß in dem Falle, in dem es ein Stoßwellengenerator ist, der Körperstein im Fokusbereich des Stoßwellengenerators liegt. Die Mittel zur richtigen Positionierung des Therapiegerätes sind dabei kostengünstig, da ein Röntgenortungssystem zur Untersuchung des Objektes normalerweise an sich vorhanden ist und zur Positionierung ein verhältnismäßig geringer zusätzlicher Aufwand erforderlich ist. Anhand des Monitors kann dabei die Sollposition mit Hilfe eines Lichtgriffels, einer Maus, eines Trackballs oder dergleichen eingegeben werden.

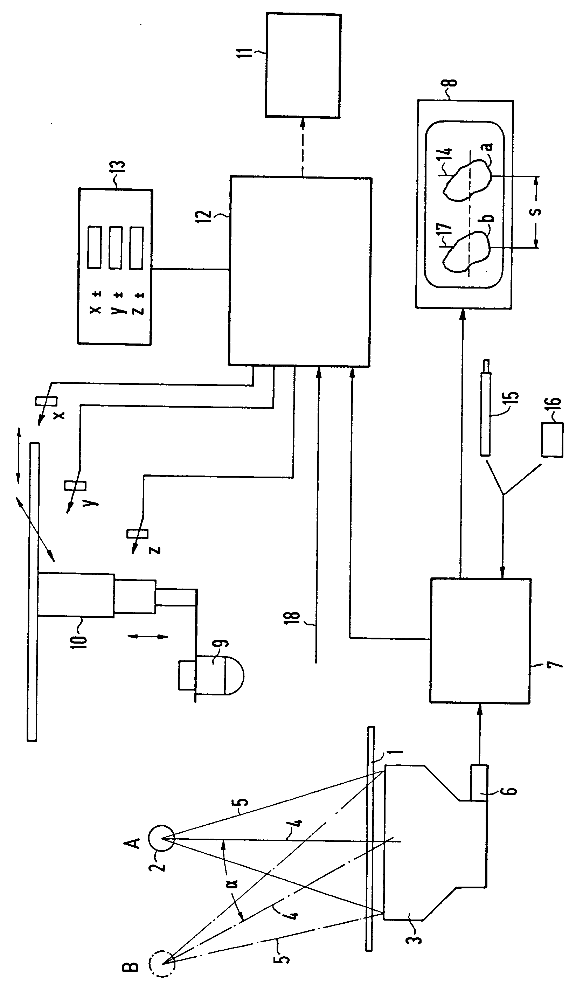

Die Erfindung ist nachfolgend anhand eines in der Zeichnung dargestellten Ausführungsbeispieles näher erläutert.

In der Zeichnung ist ein Patiententisch 1 dargestellt, auf dem ein Patient liegt, welcher von einem Röntgenortungssystem mit einer Röntgenröhre 2 und einem Röntgenbildverstärker 3 durchstrahlbar ist. Die Röntgenröhre 2 kann in zwei Positionen A und B verstellt werden, so daß der Zentralstrahl 4 des von ihr ausgesandten Röntgenstrahlenbündels 5 einen Winkel Alpha einschließt. Das Ausgangsbild des ortsfesten Röntgenbildverstärkers 3 wird von einer Fernsehkamera 6 aufgenommen und über eine Fernsehzentrale 7 mit einem Bildspeicher auf einem Monitor 8 wiedergegeben.

Zur Zertrümmerung von Körpersteinen im Patienten ist ein Stoßwellengenerator 9 vorgesehen, der über ein Stativ 10 dreidimensional an der Decke des Behandlungsraumes aufgehängt ist. Die drei Koordinaten, in denen der Stoßwellengenerator 9 verstellbar ist, sind mit x, y und z bezeichnet. Die Verstellung des Stoßwellengenerators 9 längs der Koordinaten x, y und z erfolgt durch einen Positionierantrieb 11 mit den einzelnen Koordinaten x, y und z zugeordneten Elektromotoren oder bei manueller Ausführung durch Lösen der Stativbremsen und Nullabgleich einer Differenzanzeige 13 am Anzeigedisplay.

Ein Rechner 12 vergleicht die Istposition des Stoßwellengenerators 9 (x-, y- und z-Signale) mit der anhand des Monitors 8 festgelegten Sollposition und steuert den Positionierantrieb 11 in der Weise an, daß die Istposition und die Sollposition zusammenfallen. Eine Differenzanzeige 13 zeigt dabei jeweils die Differenz zwischen der Ist- und der Sollposition an.

Die Ortung des zu behandelnden Körpersteines und die Positionierung des Stoßwellengenerators 9 erfolgt entsprechend folgendem Ablauf:
1. Der Körperstein wird bei vertikalem Strahlengang auf die im Fernsehbild auf dem Monitor 8 angezeigte Referenzmarke 14 positioniert. Er liegt dabei z.B. auf dem Zentralstrahl 4 über dem Patiententisch 1.
2. Die Röntgenröhre 2 wird um den Winkel Alpha geschwenkt.
3. Der Körperstein wandert nun im Fernsehbild auf dem Monitor 8 proportional zu seinem Weg auf dem Eingangsleuchtschirm des Röntgenbildverstärkers 3 um die Strecke s von der Position a in die Position b aus.
4. Der Körperstein wird auf dem Monitor 8 in der Position b markiert, und zwar z.B. durch einen Lichtgriffel 15, eine Maus 16, einen Trackball oder dergleichen (Marke 17).
5. Die Differenz zwischen den Positionen a entsprechend der Referenzmarke 14 und b entsprechend der Marke 17, d.h. der Weg s wird an den Rechner 12 übermittelt.
6. Im Rechner 12 wird aus dem Weg s, der Drehpunktlage für die Röntgenröhre 2, dem Winkel Alpha und dem Fokus-Bildschicht-Abstand die Höhenlage des Körpersteines zum Drehpunkt ermittelt. Zusammen mit den aktuellen Gerätepositionen - entsprechende Signale liegen am Eingang 18 - kann nun die Lage des Körpersteines im Raum bestimmt werden.
7. Vom Rechner 12 wird nun die Differenz zwischen der Position des Körpersteines und der Position des Fokusbereiches des Stoßwellengenerators 9 berechnet und an der Differenzanzeige 13 in den Raumkoordinaten x, y, z angezeigt. Ist der Positionierantrieb 11 vorhanden - dies ist nicht zwingend erforderlich -, so werden die Differenzwerte an diesen Positionierantrieb 11 weitergegeben.
8. Das Stativ 10 wird, ggf. über den Positionierantrieb 11, so verstellt, daß die Istposition des Fokusbereiches des Stoßwellengenerators 9 mit der durch die räumliche Lage des Körpersteines vorgegebenen Sollposition übereinstimmt. Der Fokusbereich und der Körperstein liegen nun an der gleichen räumlichen Stelle und der Therapiebeginn kann erfolgen.

Die beschriebene medizintechnische Anlage zeichnet sich durch einen einfachen Aufbau aus, wobei eine exakte Positionierung des Stoßwellengenerators 9 möglich ist. Das Gerät 1 bis 6 ist dabei ein Gerät, das in Verbindung mit der Fernsehzentrale 7 und dem Monitor 8 Röntgenuntersuchungen mit Schrägdurchstrahlung des Patienten ermöglicht.

Anstelle eines Stoßwellengenerators kann bei der medizintechnischen Anlage gemäß der Zeichnung auch ein anderes Therapiegerät Verwendung finden, das im Hinblick auf eine vorgegebene Stelle des Patienten positioniert werden muß. Ferner können die die räumliche Lage eines Körperbereiches wiedergebenden Informationen auch mit anderen Ortungssystemen, z.B. einem Ultraschallortungssystem, gewonnen werden.

## Patentansprüche

1. Medizintechnische Anlage mit einem Ortungssystem (2, 3, 6, 7, 8) zur Durchstrahlung des Objektes unter verschiedenen Richtungen zur Gewinnung von Informationen über die räumliche Lage von Körperbereichen sowie mit einem Therapiegerät (9), bei der eine relative Einstellung des Therapiegerätes (9) gegenüber einem Patiententisch (1) durch einen Rechner (12) in der Weise erfolgt, daß der Fokusbereich des Therapiegerätes (9) und eine Sollposition zusammenfallen, **dadurch gekennzeichnet,** daß die Sollposition des Therapiegerätes (9) in den Rechner (12) anhand von Informationen über die räumliche Lage des Körperbereiches eingebbar ist, daß das Ortungssystem nur einen Monitor (8) aufweist, dem Markierungsmittel (15, 16) für die Sollposition zugeordnet sind und auf dem bei Änderung der Strahlungsrichtung um einen Winkel Alpha der Körperbereich um eine Strecke (s) wandert, und daß der Rechner (12) so ausgebildet ist, daß er aus dieser Strecke (s), diesem Winkel Alpha und weiteren Parametern des Ortungssystems (2, 3, 6, 7, 8) die Lage des Körperbereiches im Raum ermittelt, die Differenz zur Position des Fokusbereiches berechnet und das Therapiegerät (9) im Sinne eines Ausgleiches dieser Differenz einstellt.

## Claims

1. A medical therapy system comprising a locating system (2, 3, 6, 7, 8) for irradiating an object from different directions in order to obtain data relating to the spatial position of body regions and comprising a therapy device (9) wherein a relative adjustment of the therapy device (9) in relation to a patient support (1) is effected by a computer (12) in such manner that the focal region of the therapy device (9) and a theoretical position coincide, characterised in that the theoretical position of the therapy device (9) can be input into the computer (12) in the form of data relating to the spatial position of the body region, that the locating system comprises only one monitor (8) to which marking means (15, 16) for the theoretical position are assigned and on which the body region migrates by a distance (s) in the event of a change in the direction of radiation by an angle alpha, and that the computer (12) is designed such that from this distance (s), this angle alpha, and further parameters of the locating system (2, 3, 6, 7, 8), it determines the position of the body region in space, calculates the difference in relation to the position of the focal region and adjusts the therapy device (9) in terms of compensation of this difference.

## Revendications

1. Installation médicale comportant un système de repérage (2,3,6,7,8) pour irradier l'objet dans différentes directions afin d'obtenir des informations concernant la position spatiale de parties du corps, ainsi qu'un appareil de thérapie (9), et dans laquelle un réglage relatif de l'appareil de thérapie (9) par rapport à un plateau formant couchette pour le patient (1) est réalisé au moyen d'un calculateur (12) de manière que la zone focale de l'appareil de thérapie (9) et une position de consigne coïncident, caractérisée par le fait que la position de consigne de l'appareil de thérapie (9) peut être introduite dans le calculateur (12) sur la base d'informations concernant la position spatiale de la partie du corps, que le système de repérage possède uniquement un moniteur (8), auquel sont associés des moyens (15, 16) de marquage de la position de consigne et sur lequel la partie du corps se déplace sur une distance (s) lors d'une modification de la direction du rayonnement, d'un angle alpha, et que le calculateur (12) est agencé de manière à déterminer, à partir de cette distance (s), de cet angle alpha et d'autres paramètres du système de repérage (2,3,6,7,8), la position spatiale de la partie du corps, à calculer la différence par rapport à la position de la zone focale et à régler l'appareil de thérapie (9) dans le sens d'une compensation de cette différence.
